# EUROPEAN PATENT APPLICATION

(11) **EP 4 404 208 A1**
(43) Date of publication of application: **24.07.2024**
(21) Application number: 23151896.0
(22) Date of filing: 17.01.2023
(51) Int. Cl.: G16H 40/40, G16H 40/63, G16H 50/20, H04W 4/80

(54) **MEDICAL DIAGNOSTIC DEVICE REMOTE SERVICE AND MANAGEMENT METHOD AND SYSTEM**

(71) Applicant: Esaote S.p.A., 16152 Genova (IT)
(72) Inventor: RUSSANO, Luca, 16145 GENOVA (IT)
(74) Representative: Karaghiosoff, Giorgio Alessandro

(57) **Abstract**

A medical diagnostic device remote service and management system is provided the said system comprising:
at least one medical diagnostic apparatus configured to acquire and/or process and/or store medical image data and/or medical non-image data;
at least a remote service and/or management unit including a service and/or management device for carrying out service and/or management tasks on the at least one medical diagnostic apparatus, the service and/or management tasks being carried out automatically or upon request;
a communication link being provided for the at least one medical diagnostic apparatus and the at least one remote service and management unit for exchanging data for executing the said service tasks and/or management tasks on the said at least one medical diagnostic apparatus;
the said communication link comprising:
a communication device of the at least one medical diagnostic apparatus and the communication interface of a wearable or portable processing unit being configured to communicate by means of a so called short-range wireless communication protocol;
a communication unit of the wearable or portable processing unit and of the at least one service and/or management unit being configured to communicate according to a so-called network communication protocol.

## Description

### BACKGROUND OF THE INVENTION

Medical diagnostic devices, including imaging systems, image archiving and retrieval systems, and the like are provided in combination with network connections that permit remote field service requests to be generated and communicated to a service center, and therethrough to be relayed to the networked diagnostic devices.

The term Medical diagnostic devices include in a non-exhaustive list thereof magnetic resonance imaging (MRI) systems, computed tomography (CT) imaging systems, conventional and digital x-ray systems, positron emission tomography (PET) systems, ultrasound systems, and so forth.

In combination with the medical diagnostic devices, such as the above listed diagnostic image acquisition devices there are often provided also other diagnostic equipment such as picture archives and communication systems (called briefly PACS) for storing digitized images and for retrieving and communicating the images to receiving units. The said one or more diagnostic imaging devices and the said one or more PACS stations may be networked to fully integrate their work flow.

In the technical field it is customary to provide highly trained service personnel for monitoring operation of the diagnostic systems and carrying out services intervention on hardware, firmware, and software of the devices and systems, to maintain proper and full range operativity and to carry out upgrades and/or integration of new or improved software tools, for example for image acquisition and/or image processing. In relation to the above service and /or management operations field service technicians are able to carry out the said operations or at least some steps of the said operations remotely, through network connections with the diagnostic imaging devices and the diagnostic systems provided in combination therewith.

Remote servicing and/or management of medical diagnostic systems, can be placed in direct communication with remote service centres, such as via an open network connection or a virtual proprietary network. Such service centres can be provided at the production site of the said diagnostic or medical systems and/or also at the site of third parties which operate as a service provider which is external to the organization of the system producers.

Currently the connections between the medical and/or diagnostic devices occurs through a traditional Lan network using known protocols such as TCP/IP communication protocols and connects directly to the service and management system or through a portal which can be accessed through internet.

EP0795295 discloses a method for remotely upgrading a medical diagnostic ultrasound system by the addition of new or different performances features and functions, said method comprising the steps of:
- storing configuration information of said ultrasound system,
- transmitting upon electronic request from a remote location said configuration information over a common carrier communications medium to said remote location,
- receiving an ultrasound upgrade program data transmitted from the remote location over the common carrier communications medium, on the basis of the configuration information, and
- installing the ultrasound upgrade program data in the ultrasound system.

The communications link may take the form of a network interface such as an Ethernet port which communicates through a network in the facility where the ultrasound system resides, or it may take the form of a modem which interfaces to telephone lines. In either case the communications link is capable of communicating with and receiving program data transmitted to the ultrasound system from the factory. The communications link is controlled within the ultrasound system by the processor and program data received by the communications link from the factory may be processed by the processor or stored in the data storage media, or both.

US5851186 discloses a medical ultrasonic diagnostic imaging system which is capable of being accessed over data communication networks such as the Internet, making the ultrasonic images, diagnostic reports, and ultrasound system diagnostics information and operation accessible to a conventional personal computer using commercially available software at virtually any remote location. In one embodiment, the ultrasound system can be remotely operated from the personal computer. The inventive apparatus and techniques make it possible for physicians to remotely access, control, and perform diagnoses using their ultrasound systems over a network such as the World Wide Web with no special hardware requirements.

US2003014425 discloses diagnostic systems which are accessible by a service facility through network connections of various types, including open and proprietary networks. A field service technician is equipped with an interface unit, such as a laptop computer, through which service request modules may be identified and transmitted to the service facility, such as through an electronic message. The service request modules may include a wide variety of standard requests, depending upon the specific system kind and configuration. The service technician may select the service request from a menu, thereby alleviating the need to predefine or memorize available standard requests. In response to the message from the field service technician, the service facility contacts the identified diagnostic system and accesses the information required by the service technician. This process may be fully automated to expedite handling without human intervention. Data or reports may then be compiled and re-transmitted to the service technician via electronic messages in response to the request. Data may be exchanged between the diagnostic systems, field service units, and remote service facility in any suitable format, such as in accordance with the Internet Protocol (IP), the Transmission Control Protocol (TCP), or other known protocols. Moreover, certain of the data may be transmitted or formatted via markup languages such as the HyperText Markup Language (HTML), or other standard languages.

In the communication methods and systems according to the state of the art, for example the above mentioned documents, the communication between the remote service and/or management unit, such as a server or the like, are configured according to the well known and widely used IP or TCP or similar protocols, requiring relatively complex and time consuming connection procedures as well as the need of remembering access credentials such as a user ID and a password or similar access keys in order to setup the communication link between a medical device, such as an imaging apparatus and a remote service and/or management station.

Using one of the above-mentioned communication modalities for the remote connection between user device such as a diagnostic imaging apparatus, for example an ultrasound imaging apparatus and the factory service centre or the service centre of a third party service provider allows to download log data for diagnostic purposes, while physical presence of Service Personnel is required to upload new settings/licenses etc.

The above communication modalities according to the state of the art has several drawbacks, some of which are schematically listed in the non exhaustive following list:
Temporal constraint:
   User and Esaote service need to be connected at the same time;
Connectivity constraint:
   dedicated internet connection and configuration need to be available meanwhile Service provides and User communicate.

Need of physical presence of Service personnel for advanced operations.

Since the above service and management operations are crucial for the maintenance of the performances of the medical devices and the number of medical devices and the number of accesses to the remote service and management stations increases, improvements of the current communication links and methods between the medical devices and the remote service and/or management stations would be welcome. This could help in facilitating the connections, facilitating the tasks carried out by the service persons and reducing time and costs for service and/or monitoring of operative conditions and/or managing medical devices and particularly diagnostic imaging devices.

### SUMMARY OF THE INVENTION

According to a first aspect, a medical diagnostic device remote service and management system is provided the said system comprising:
at least one medical diagnostic apparatus configured to acquire and/or process and/or store medical image data and/or medical non-image data;
at least a remote service and/or management unit including a service and/or management device for carrying out service and/or management tasks on the at least one medical diagnostic apparatus, the service and/or management tasks being carried out automatically or upon request;
a communication link being provided for the at least one medical diagnostic apparatus and the at least one remote service and management unit for exchanging the data for starting a connection between the said at least one medical diagnostic apparatus and the said at least one remote service and/or management unit and for exchanging data for executing the said service tasks and/or management tasks on the said at least one medical diagnostic apparatus;
the said communication link comprising:
   a communication device at each of the said medical diagnostic apparatus;
   at least one wearable or portable processing unit for a corresponding user or service person, the said at least one processing unit being provided with a communication interface, configured to communicate with the communication device of said at least one medical diagnostic apparatus;
   the communication device of the at least one medical diagnostic apparatus and the communication interface of the wearable or portable processing unit being configured to communicate by means of a according to a so called short-range wireless communication protocol;
   the wearable or portable processing unit comprising further a communication unit configured to communicate with a communication unit of the at least one remote service and/or management unit,
   the said communication unit of the wearable or portable processing unit and of the at least one service and/or management unit being configured to communicate according to a so-called network communication protocol.

With the term short range wireless communication protocols the present invention refers to systems providing wireless connectivity within a local sphere of interaction.

A non-exhaustive and not limiting list of known short range wireless communication systems and protocols comprises for example the following protocols: Bluetooth, Wi-Fi, ZigBee, UWB, IR

The term network protocols encompass so-called network management protocols and/or network communication protocols and/or network security protocols.

With the term Network Communication Protocols communication protocols are intended which are vital to the functioning of a network. In fact, computer networks can't exist without these protocols. These protocols formally describe the formats and rules by which data is transferred over the network. This is a must-have for exchanging messages between your computing systems and in telecommunications, applying to both hardware and software. Communication protocols also handle authentication and error detection as well as the syntax, synchronization and semantics that both analog and digital communications must abide by to function.

The following is a non exhaustive exemplary list of network communication protocols according to the above definition:

HTTP-hyper text transfer protocol (HTTP)- is often referred to as the protocol of the internet. HTTP is an application layer protocol that allows the browser and server to communicate.

TCP -Transmission Control Protocol (TCP)-separates data into packets that can be shared over a network. These packets can then be sent by devices like switches and routers to the designated targets.

UDP -User Datagram Protocol (UDP)- works in a similar way to TCP, sending packets of data over the network.

IRC - Internet Relay Chat (IRC) is a text-based communication protocol. Software clients are used to communicate with servers and send messages to other clients. This protocol works well on networks with a large number of distributed machines.

In the present disclosure, and in the claims the term service comprises different operations which can be carried out on the said at least one medical diagnostic device, such as for example, monitoring operational conditions of the medical diagnostic device, looking for conditions in the operational status of the device which are or may lead to misfunctions, determining the interventions to be carried out for fixing the said malfunctional operative conditions, carrying out the said interventions and/or instruction service persons to carry out the said interventions, suggesting upgrades of the device and/or carrying out the said upgrades and/or instructing service persons to carry out the said upgrades, selecting, ordering and/or purchasing and/or downloading from the said at least remote service and/or management station software to be installed on the said medical diagnostic device.

The above list is only exemplary and not exhaustive. Furthermore service may consist in interventions to be carried out on the hardware which requires that a service person physically interacts with the hardware of the medical diagnostic device. Alternatively service interventions may consist in redefining the device settings, and/or fixing software problems and/or installing software patches or software upgrades.

In relation to the meaning of the term management, the remote service and/or management station directly or through the wearable or portable device and/or the wearable or portable device directly may be configured in order to take over the control of the medical diagnostic device in place of the control console being part of the said medical diagnostic device.

Alternatively or in combination the management may include also tasks relating to processing the acquired images in order to carry out computer aided image enhancing and or interpretation and or diagnostic processes and/or also in order to merely improve the image quality by means of digital image processing algorithms.

Also in relation to the term management the list of possible operation and tasks is only exemplary and not exhaustive and also not limiting.

According to an embodiment, the communication protocols between the wearable and/or portable unit and the remote service and/or management station are configured in such a way that a user ID and a user password are requested for allowing the user of the wearable and/or portable unit to connect the said unit with the remote service and/or management station and to access the possible service and/or management options provided by the said remote service and/or management station.

User ID and password may be double, one relating to the enabling of the wearable and/or portable device to connect to the remote service and/or management station. A further user ID and password may be also provided which relates to the enabling the person using the wearable and/or portable unit to access the said unit and/or also to be allowed to carry out one or only some or all of the service options and/or of the management options available through the remote service and/or management station.

According to this provision a double check of the fact that the user connecting to the remote service and/or management station is effectively authorised to connect at all and to select and carry out certain specific services operations and or management operations.

According to a further embodiment the connection between the wearable and/or portable unit and a medical diagnostic device may also be managed by means of access credentials such as univocal device ID.

This univocal device ID may be saved in a memory in the control hardware of the medical diagnostic device and be automatically sent or made readable to the wearable and/or portable device, or the said univocal device ID may be in ethe form of a graphical and or alphanumerical code.

According to an embodiment, the wearable and/or portable unit comprises a processor, memories for storing the control software of the wearable or portable unit itself and the software for carrying out the communication processes with the medical diagnostic devices and with the remote service and/or management station, a user interface for inputting selections and or command, a display interface for showing alphanumeric messages and/or images, a camera for acquiring images and optionally also at least one loudspeaker and/or a microphone.

In the case of providing device ID in the form of graphic codes, such as bar codes and/or QR codes, the wearable and/or portable unit may read an image of the said graphic codes and extract the information coded therein, basically but not only the said univocal device code.

The wearable and/or portable unit can be in the form of a PDA, a table or a smartphone or a similar device.

According to an embodiment, in the wearable and/or portable device an APP is saved and installed, in which up the instruction are coded to the hardware of the said wearable and/or portable device in order to carry out the connections to the medical diagnostic device and to the remote service and/or management station and to access the functions and tasks for servicing and for managing the said medical diagnostic device made available by the remote service and management station for the specific wearable and/or portable unit and/or for the user of the said unit and/or for the medical diagnostic device identified by the univocal device ID.

As it appears clearly from the above features, the conventional network connection to the remote service and/or management station is carried out by the wearable and/or portable device. No direct connection of the medical diagnostic device to the remote service and/or management station is provided and the communication link between the medical diagnostic device and the wearable and/or portable device occurs by means of the simpler short range communication protocols which overcomes the complexity of the settings of the traditional network communication.

In some way the wearable and/or portable device is an intermediary between medical diagnostic devices and remote service and/or management servers. In some way but not limiting it is a sort of gateway between the short range communication network between medical diagnostic devices and wearable and/or portable unit and the network communication system between the said wearable and/or portable unit and the said remote service and/or management station. This avoids the necessity to provide each medical device with the hardware and software for connecting to a network communication system as defined above and limits hardware and software to the need of the communication hardware and software for carrying out short range Wi-Fi communications which are fare simpler than the network communication systems.

### BRIEF DESCRIPTION OF THE DRAWINGS

Figure 1 shows a high-level diagram of an embodiment of an ultrasound system as an example of a medical diagnostic device.
Figure 2 shows a diagram of an embodiment of a wearable and/or portable device.
Figure 3 shows an embodiment of the system according to the present invention in which the medical device is an ultrasound apparatus and the wearable or portable device is a smartphone.
Figure 4 is an embodiment showing the use of the system according to figure 3 for carrying out sharing of diagnostic images in a videoconference with other parties.
Figure 5 is a generic workflow diagram of a system according to the present invention.
Figure 6 is a workflow diagram illustrating the steps for carrying out a videoconference sharing the output data of the medical device.
Figure 7 is a workflow diagram illustrating the steps for carrying out a download of upgrades of the medical device from an online shop.

### DETAILLED DESCRIPTION OF THE DRAWINGS

In the following description the medical device disclosed is an ultrasound imaging system. The said ultrasound imaging system is to be intended only as a non-limiting example. Instead of the ultrasound imaging system any kind of other medical devices can be provided, particularly of devices which have an hardware comprising a control processing unit capable of controlling a communication unit and also capable of carrying out additional software relatively to the one specifically needed for controlling the functions for which the medical device is configured.

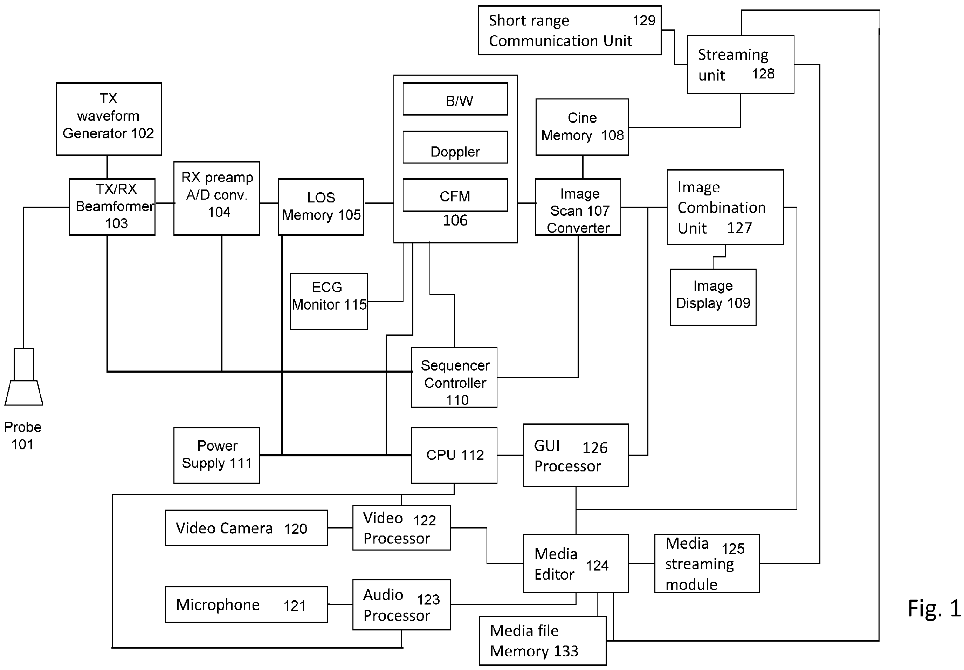

Fig. 1 illustrates a high-level block diagram of an ultrasound system according to an embodiment. Portions of the system (as defined by various functional blocks) may be implemented with dedicated hardware, such as transmit/receive (TX/RX) driving/preamp and power switching circuitry, which may utilize analog components. Digital components, DSPs and/or FPGAs, may be utilized to implement the sequencer controller and the timing generator.

The ultrasound system of Fig. 1 includes one or more ultrasound probes 101. The probe 101 may include various transducer array configurations, such as a one-dimensional array, a two-dimensional array, a linear array, a convex array and the like. The transducers of the array may be managed to operate as a 1D array, 1.25D array, 1.5D array, 1.75D array, 2D array, 3D array, 4D array, etc.

The ultrasound probe 101 is coupled over a wired or wireless link to a beamformer 103. The beamformer 103 includes a transmit (TX) beamformer and a receive (RX) beamformer that are jointly represented by TX/RX beamformer 103. The beamformer 103 supplies transmit signals to the probe 101 and performs beamforming of "echo" signals that are received by the probe 101.

A TX waveform generator 102 is coupled to the beamformer 103 and generates the transmit signals that are supplied from the beamformer 103 to the probe 101. The transmit signals may represent various types of ultrasound TX signals such as used in connection with B-mode imaging, color Doppler imaging, pulse-inversion transmit techniques, contrast-based imaging, M-mode imaging and the like. The beamformer 103 performs beamforming upon received echo signals to form beamformed echo signals in connection pixel locations distributed across the region of interest. For example, in accordance with certain embodiments, the transducer elements generate raw analog receive signals that are supplied to the beamformer. The beamformer adjusts the delays to focus the receive signal along a select receive beam and at a select depth within the ROI. The beamformer adjusts the weighting of the receive signals to obtain a desired apodization and profile. The beamformer sums the delayed, weighted receive signals to form RF beamformed signals. The RF beamformed signals are digitized at a select sampling rate by the RX preamp and A/D converter 104. The RF beamformed signals are converted to I,Q data pairs.

The I,Q data pairs are saved as image pixels in the line of sight (LOS) memory. For example, the LOS memory may include LOS memory portions associated with each line of sight through the ROI. The I,Q data pairs, defining the image pixels for corresponding individual ROI locations along a corresponding LOS, are saved in the corresponding LOS memory portion. A collection of image pixels (e.g., I,Q data pairs) are collected over time and saved in the LOS memory 105. The image pixels correspond to tissue and other anatomy within the ROI.

In embodiments, a dedicated sequencer/timing controller 110 may be programmed to manage acquisition timing which can be generalized as a sequence of firings. The sequence controller 110 manages operation of the TX/RX beamformer 103 and the A/D converter 104.

One or more processors 106 perform various processing operations as described herein. The CPU 112 may perform control operations of various units such as the processors 106, the GUI-image processor 162, the video processor 122 and the sound or audio processor 123.

Among other things, the processor 106 and/or CPU 112 analyse the image pixels to determine differences between each following image from a preceding one of a time sequence of images and to control the video processors and/or the media editor or the streaming module 125 in order to discard from the data stream images which are identical or almost identical to the previous one by maintaining on screen the previous image.

The processor 106 and/or CPU 112 also performs conventional ultrasound operations. For example, the processor 106 executes a B/W module to generate B-mode images. The processor 106 and/or CPU 112 executes a Doppler module to generate Doppler images. The processor executes a Color flow module (CFM) to generate color flow images. The processor 106 and/or CPU 112 may implement additional ultrasound imaging and measurement operations. Optionally, the processor 106 and/or CPU 112 may filter the displacements to eliminate movement-related artifacts.

An image scan converter 107 performs scan conversion on the image pixels to convert the format of the image pixels from the coordinate system of the ultrasound acquisition signal path (e.g., the beamformer, etc.) and the coordinate system of the display. For example, the scan converter 107 may convert the image pixels from polar coordinates to Cartesian coordinates for image frames.

A cine memory 108 stores a collection of image frames over time. The image frames may be stored formatted in polar coordinates, Cartesian coordinates or another coordinate system.

An image display 109 displays various ultrasound information, such as the image frames and information measured in accordance with embodiments herein. The display 109 displays the ultrasound image with the region of interest shown. Optionally, the system of Fig. 1 may include an ECG monitor not shown that couples an ECG sensor to the patient and records an ECG signal indicative of the patient's heart rate. The processor 106 and/or sequence controller 110 synchronize the image acquisition steps with the ECG signal.

The blocks/modules illustrated in Fig. 1 can be implemented with dedicated hardware (DPSs, FPGAs, memories) and/or in software with one or more processors.

A control CPU module 112 is configured to perform various tasks such as implementing the user/interface and overall system configuration/control. In case of fully software implementation of the ultrasound signal path, the processing node usually hosts also the functions of the control CPU.

A power supply circuit 111 is provided to supply power to the various circuits, modules, processors, memory components, and the like. The power front-end may be an A.C. power source and/or a battery power source (e.g., in connection with portable operation).

According to a further feature an image combination unit 127 may be present in which the B-mode image data of at least of a region of interest and the corresponding graphic representation of the GUI and further textual information associated to the image acquisition such as data of the patient, indication of the diagnosis, setting of the scanner is combined for the superimposed display of the B-mode image and of the said data.

The acquired images as well as the GUI images and the textual data are combined in the Image combination unit and the combined images are displayed on the image display of the ultrasound system.

The said acquired images and the GUI images and the textual data are also processed by a media editor 124. The media editor 124 is configured to combine the acquired images, the images of the GUI and the textual data as well as other imaged data such as acoustic data and further video images in order to generate a multimedia video clip coded as a multimedia file according to one or more of the current available coding protocols for video and acoustic files.

GUI processor 126 is also configured to generate a graphic code, such as a bar code or a QR code in which information can be coded such as an ID code of the ultrasound system and further other information as for example information to access streaming data from the image display screen 109. By reading the code a device can retrieve the information necessary to start a streaming connection with the ultrasound apparatus.

Acoustic data may be generated by the ultrasound system such as the audio files representing the hematic flows in the various flow imaging modes such as Doppler, power Doppler, etc.. According to a variant shown in figure 1, the acoustic data can be generated by a microphone 121 capturing noises or speeches held by one or more of the operators using the ultrasound system. Microphone signals are fed to an audio processor 123 which is configured to code the acoustic signals in audio files. The audio files are fed to the media editor for being associated to one or more of the further images in a synchronized manner with the content of at least one of the said images.

Image data may be captured by a camera 120 reproducing the ultrasound system or part of it, the patient or an anatomic district of a patient and at least one of the operators of the ultrasound system. The image data acquired by the camera is processed by a video processor 122 and also fed to the media editor for being combined in a multimedia representation with the audio files and/or with one or more of the acquired images and/or with the GUI images and/or with the images reproducing textual or alphanumeric coded data.

The media editor 124 may be configured by settings of the user and/or by factory pre-sets to combine the image information and the audio information according to various schemes generating a common multimedia file or the acquired images and/or the GUI images and/or the images reproducing textual or alphanumeric data, the one or more audio files are made available as separate media files which can be downloaded and displayed in parallel in different areas of the display of a client and in a synchronized manner at least for some of the said images and or audio files.

The multimedia files generated by the media editor are fed to a media streaming module 125 which can be accessed through a video and audio streaming unit 128 by a client connecting to a communication unit or port 129 of the ultrasound system.

The client is configured to execute a connection using a short-range communication protocol according to one or more of the above listed exemplary embodiments which allows access to the multimedia content.

The media editor 124 may also be configured to render part of the visual and or acoustic information not available by covering the display areas where this information is printed on the display screen by a banner and by silencing the audio files or part of them.

According to an embodiment the multimedia files generated by the media editor 124 may be stored in a media file memory 133 and the access to the said file by means of the media streaming module 125 can be executed in real-time or at a later time in relation to the time of generation of the visual and/or acoustic information with an off-line streaming process.

The different units and processors 122, 123, 124, 125, 126, 128 may consist all or a part of them in dedicated hardware.

In an alternative embodiment the said units and processors may consist at least for a part of them by a dedicated generic processing unit executing a software program in which the instructions are coded for configuring the generic processing unit in carrying out the functions of the specific processor and/or unit 122, 123, 124, 125, 126, 128.

In a further variant embodiment at least part of the said units and processors may consist in a software which is executed by the CPU 112 configuring the said CPU and its peripherals for executing the functions of the said at least part of the units and processors 122, 123, 124, 125, 126, 128.

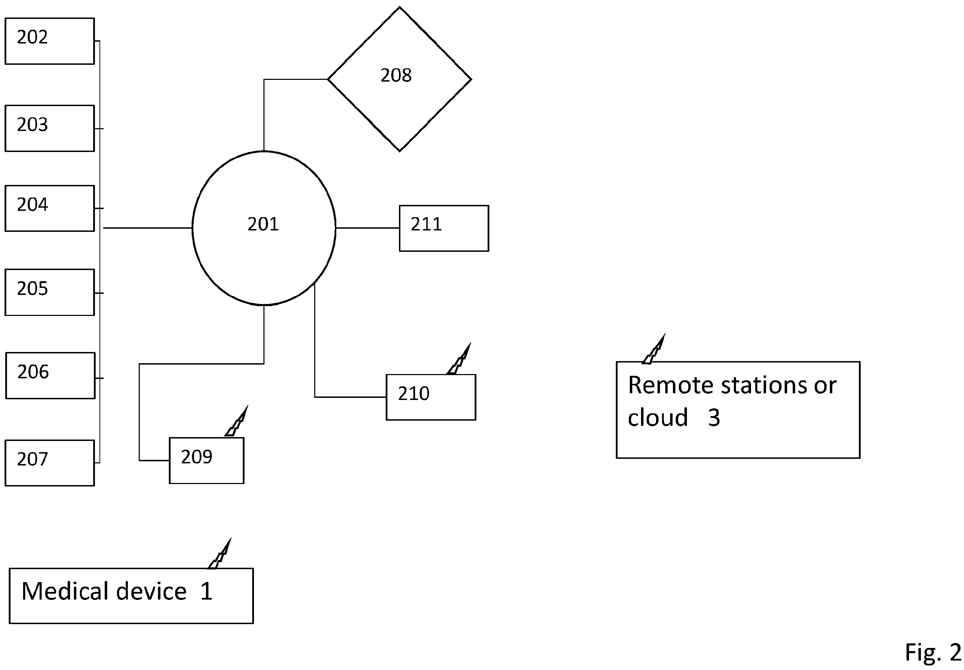

Figure 2 shows an example of a generic hardware of a portable and/or wearable unit which is associated to one user which is responsible for using the medical imaging device and/or for carrying out service interventions on it.

The portable or wearable unit 2 comprises a processing unit 201. One or more user interfaces are connected as peripherals to the processing unit.

As a non limiting and non exhaustive example the said user interfaces may comprise in any combination one or more of the following devices:
a display 202, a keyboard 203, a mouse or similar pointing and selection device 204, a microphone 205, a loudspeaker 206, a video or photo camera 207.

Some of the above interfaces may be integrated in a combination of a touch-screen display and a software controlling the said touch-screen for carrying out the functions of different interface types, such as for example the functions of the display, of the keyboard and of the pointing and selection device.

A memory 208 is provided for saving software programs which comprises the instructions for controlling the functions of the portable/wearable device and of the peripherals connected to it and for executing functions and operations which will be described with more detail in the following.

Furthermore, the wearable and/or portable unit 2 comprises a first communication unit 209 which is configured to operate according to so-called short-range Wi-Fi communication protocols, such as Bluetooth, Wi-Fi, ZigBee, UWB, IR or other similar communication protocols.

A second communication unit 210 is also provided which is configured to operate according to the so called network communication protocols, such as for example network management protocols and/or network communication protocols and/or network security protocols like for example:

HTTP-hyper text transfer protocol (HTTP)- is often referred to as the protocol of the internet. HTTP is an application layer protocol that allows the browser and server to communicate.

TCP -Transmission Control Protocol (TCP)-separates data into packets that can be shared over a network. These packets can then be sent by devices like switches and routers to the designated targets.

UDP -User Datagram Protocol (UDP)- works in a similar way to TCP, sending packets of data over the network.

IRC - Internet Relay Chat (IRC) is a text-based communication protocol. Software clients are used to communicate with servers and send messages to other clients. This protocol works well on networks with a large number of distributed machines.

An electric power source, such as a rechargeable accumulator or battery 211 provides for energizing the portable and wearable device 2.

Many different devices may be used as portable and/or wearable device with which a medical device user and/or service person is equipped. Particularly suitable devices are Tablets, PDA, Notebooks and Smartphones. All these devices normally are natively equipped with the above generically disclosed hardware. In particular all of these devices are natively provided with communication units configured to connect to other devices by means of one or more short-range wi-fi protocols and also with communication units capable of connecting to remote devices by means of network communication protocols according to the definition of the said protocols made in the previous disclosure. Both short-range communication protocols and network communication protocols are executed using non wired connections. The communication by means of the network communication protocols can be carried out using the cellular telephone network.

According to the present invention the portable and/or wearable unit 2 is configured to be the connection node between two worlds represented on one side by the software controlling the medical device 2 and on the other side the software controlling IT functions such as remote servicing, remote monitoring, remote managing and/or remote repairing or maintenance and/or remote upgrading functions. Furthermore, the portable and/or wearable device may also play a role as a node between the software controlling and managing the medical device 1 and the output data of the said device and remote user by allowing to access through the portable and/or wearable unit 2 videoconference platforms for sharing the data provided by the medical device 1 with other user.

Connection between the portable and/or wearable unit 2 and the medical device 1 can be rapidly and easily provided using the short-range wi-fi communication protocols, and the communication software can also be setup in such a way to render some data available by the portable and/or wearable unit 2.

In the memory of the wearable and/or portable unit 2 on the other hand applications may be stored which allow to carry out different tasks when run on the said wearable and/or portable unit 2.

The application software installed in the portable and/or wearable unit 2 may comprise instructions in order to carry out the control of the authorisation credentials of the user and/or of the unit 2, such as the user ID or the unit 2 ID and/or the corresponding passwords. Based on this authorisation credentials one or more options of service and/or management interventions and/or operations can be made available to the user at the portable and/or wearable unit 2.

As it will be described in the following examples the control of the authorisation credentials and the availability to the user through the wearable and/or portable unit 2 is carried out by a remote service and/or management station 3 such as a remote server and/or a group of remote servers and/or a cloud server or other remote processing stations.

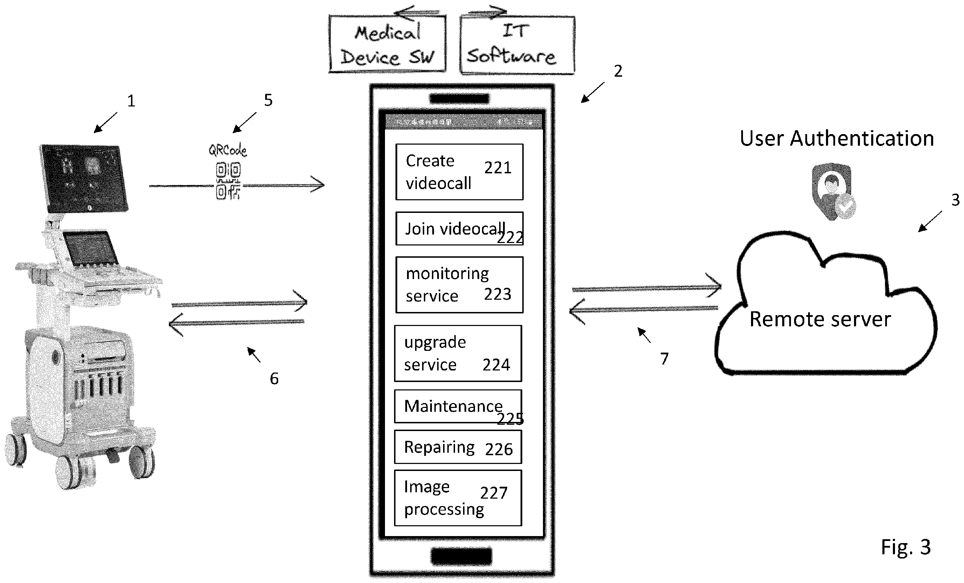

Figure 3 shows an example in which the portable and/or wearable unit 2 is a smartphone. The smartphone executes application for connecting to a remote service or processing station 3.

According to a feature of the embodiment of figure 3, the smartphone 2 executes and application in which a software is coded for reading data from the medical device 1 which are represented by the medical device coded in a graphic form such as a QR code 5 or similar. The graphic code 5 may be also in other form than a QR-code such as for example a bar code.

IN one embodiment the said graphic code is printed in a stable way on the case of the medical device 1 such that the said code may be read and also processed by the smartphone by means of its native camera and of the graphic code encrypting software loaded and executed by the smartphone 2.

In a variant embodiment the said graphic code may be generated by the medical device through a graphic code, generating software which is loaded and executed by the processing unit of the medical device and the said graphic code is printed on the display of the said medical device 1. This embodiment allows to modify the information transmitted to the smartphone 2 by means of the graphic code allowing to adapt the data to be transferred to the smartphone to different operative conditions or to make available to the smartphone 2 different data. It is also to be considered that graphic code generating software may be executed also by older devices which may have a display. This allows to transmit to the smartphone any kind of data available at the medical device, even if the said medical device does not have an interface for connecting to the hardware generating the said data in order to directly receive the signals coding the said data.

Furthermore, alternatively or in combination, as shown by the arrows 6 a two ways communication wireless channel is opened between the medical device and the smartphone 2. This channel allows to exchange data between the smartphone 2 and the medical device 1.

The said data can be of any kind such as for example the output data of the medical device, for example in this case the images or the image data, and/or settings of the medical device 1 and/or to transfer commands to the medical device 1 in order to change settings or carry out other interventions. A further possibility is also to load and install additional software for upgrades or integration of new services on the medical device 1. The said additional software can be downloaded by the smartphone from the remote service and/or management station 3 according to the options which can be made available to the user and/or to the smartphone 2 univocally identified by the user ID and password validated by the remote station 3.

In the specific example of figure 3, the smartphone 2 communicates with the remote server 3 by means of a network communication protocol and over the cellular phone network. The two way communication channel is represented by the arrows 7.

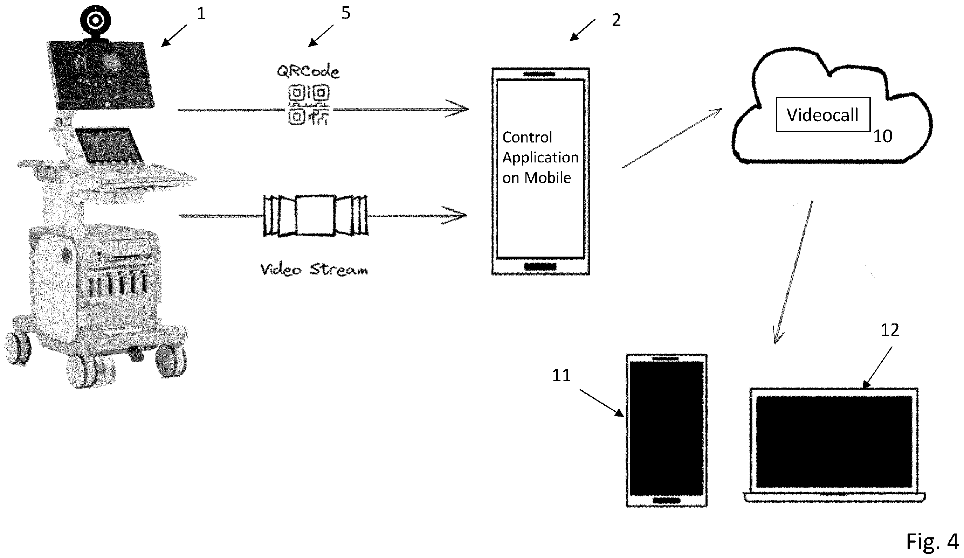

In a possible embodiment the application software executed by the smartphone sends the credentials of the user And/or of the smartphone and/or of the medical device to the remote server 3. The remote server 3 carry out the user authentication and allows access to the application on the smartphone to several services and or management operations which are available for the specific user and/or medical device. This may result in displaying on the screen of the smartphone several graphic buttons each one for launching a service operation and/or a management operation. In figure 3 an exemplary and non-exhaustive example of buttons is shown. Button 221 allows to launch through the smartphone 2 a videocall meeting in which images or output data of the medical device 1 can be shared by different persons as it is more specifically represented in figure 4. Button 222 allows to join an already launched video meeting. Button 223 starts operations relating the monitoring of the operations of the medical device 1, for example in order to identify malfunctions. Button 224 launches upgrade services by which new or improved software may be downloaded and installed on the medical device. The software is downloaded on the memory of the smartphone, from which it is further downloaded in the medical device. Control of the installation process may be taken over by the smartphone 2.

Button 225 starts maintenance interventions, button 226 launches repair interventions. Button 227 allows to download from the medical device image data or other output data and transmit these data to a remote processing station which is configured for carrying out processing algorithms on the output data of the medical device such as image processing algorithms of the image data generated by the medical device for extracting information and/or for improving image quality.

The above list is not exhaustive and in response to selecting and pressing a button of the ones disclosed above a further menu may be displayed on the display of the smartphone 2 through which further options will be made available to the user of the smartphone 2.

Figure 4 represents the situation in which through the smartphone 2 by executing a corresponding control application a videocall or meeting 10 is started or is joined in which output data, in this example images generated by the medical device 1 can be shared with other users represented by their personal devices 11, 12. The images generated by the medical device 1 are transmitted to the smartphone 2 according to a video streaming protocol indicated with 13. Possible additional data may be added to the images shared by video streaming by means of the coding of the said data in graphic codes such as the QR-code indicated by 14.

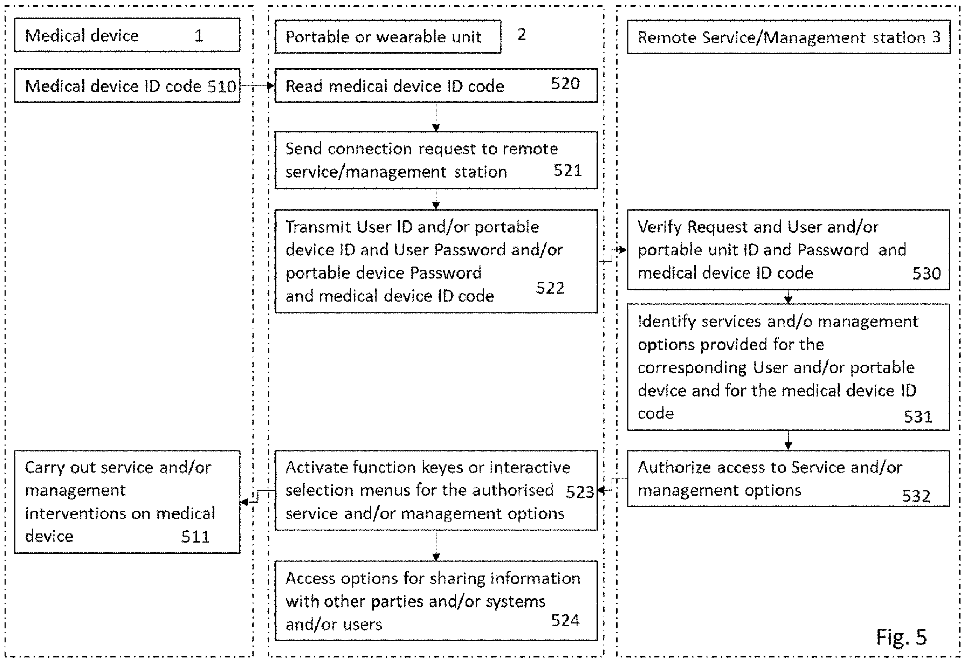

Figure 5 is a flow diagram representing an embodiment of a generic method of operation of the above-described systems. The three entities are indicated by the same numerals as in the previous figures i.e. the medical device with 1, the portable or wearable unit with 2 and the remote service and/or management station with 3. In the column above the steps carried out by the corresponding entity are described.

At step 510 the medical device makes available its ID code to the portable and/or wearable unit 2. This code, such as a graphic code disclosed in the previous embodiments is read by the portable and/or wearable unit as indicated at 520. At step 521, the portable and/or wearable unit 2 sends a connection request to the remote service and/or management station 3. Together with the said connection request also step 522 is carried out consisting in transmitting the User ID and/or the ID of the portable device and the User Password and/or the password for the portable device to the remote service and/or management station 3.

Following this step 523 at step 530 the remote service and/or management station 3 carry out an authentication process consisting for example in verifying the communication request and the received ID of the user and/or of the portable unit and the corresponding passwords.

According to a possible embodiment to each user ID or to each portable unit ID a certain list of different available services and or management options can be saved in a memory of the remote service and/or management station 3. In this case, the remote service and/or management station 3 may carry out a process for identifying services and/o management options provided for the corresponding user and/or portable device and for the medical device ID code as shown by the step 531. At step 532 the authorisations for accessing the said services and/or the said management options identified in the previous step 531 are generated and transmitted to the portable and/or wearable device 2.

In response to this the portable and/or wearable unit 2 activates the function keys or interactive selection menus for the authorised service and/or management options as indicated by step 523.

BY using the said function keys on the portable and/or wearable unit 2, the corresponding service and/or management interventions on medical device 1 as indicated by step 511.

As indicated by step 524, the portable and/or wearable unit 2 may also be authorised to access further options such as the sharing information with other parties and/or systems and/or users for examp0le by means of video meetings.

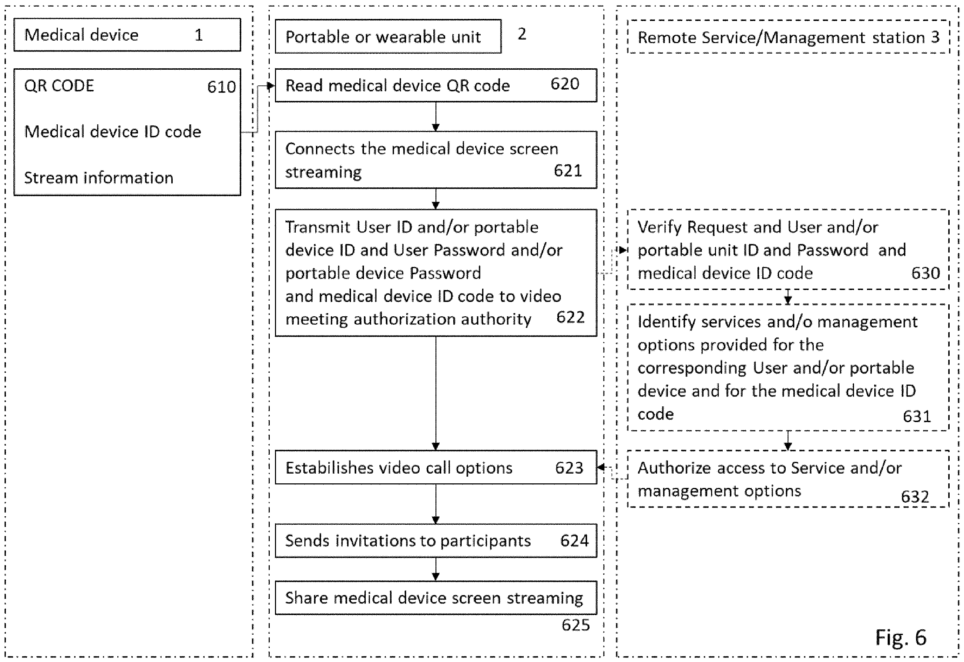

Figure 6 shows a different embodiment than the more general one of figure 5. Here as it is highlighted by the completely dotted lines of the column relating to the steps executed at the remote service and/or management stations 3, the said stations may or may not be part of the process steps.

In this case the diagram shows the steps for sharing information of a medical device 1 with other persons and/or users which are at remote places and are provided with personal devices like the one indicated with 11 and 12 in figure 4.

At step 610 the medical device 1 provides for the information relating to its ID and to the video streaming information necessary for the portable and/or wearable unit 2 to access the images generated by the medical device or other outputs. At step 620 the portable and/or wearable unit 2 reads the QR code. And at step 621 the said portable and/or wearable unit connects to the medical device screen streaming.

At step 622, the portable and/or wearable unit 2 transmits user ID and/or the device ID of the portable unit 2 and the corresponding passwords and/or also the medical device ID code to video a meeting authorization authority. This authority may be an authority managing one of the publicly accessible video meeting platforms such as for example the platform called Zoom^{®} od Temas^{®} or others, or it can be a specific dedicated and non-public meeting platform managed by the remote service and/or management station 3 as indicated by the dotted line and by the steps 630, 631 and 632.

Following authorisation by one of the two alternatives at step 623 video call options are made available by means of selection buttons on the portable and/or wearable unit 2 and following the selection of one or more of the said options the invitations of the video call are transmitted by the portable and/or wearable unit as indicated by step 624 and at step 625 also output data of the medical device 1 such as images are shared with the participants.

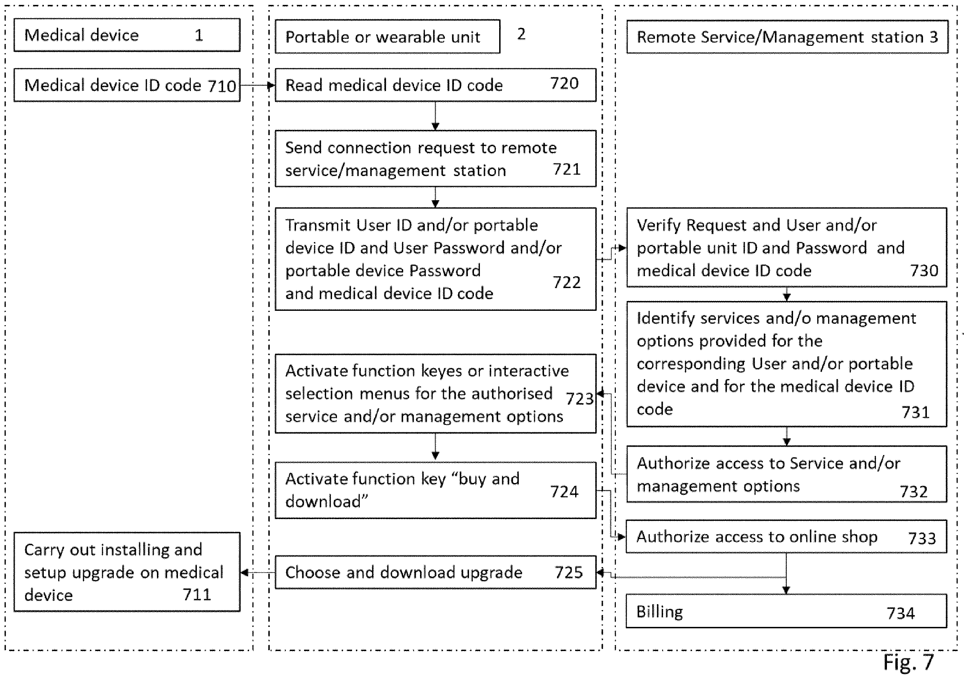

Figure 7 shows a further embodiment in which the system is used for buying and installing upgrade software on a medical device 1. At step 710 the medical device makes available its ID code to the portable and/or wearable unit 2. This code, such as a graphic code disclosed in the previous embodiments is read by the portable and/or wearable unit as indicated at 720. At step 721, the portable and/or wearable unit 2 sends a connection request to the remote service and/or management station 3. Together with the said connection request also step 722 is carried out consisting in transmitting the User ID and/or the ID of the portable device and the User Password and/or the password for the portable device to the remote service and/or management station 3.

Following this step 723 at step 730 the remote service and/or management station 3 carry out an authentication process consisting for example in verifying the communication request and the received ID of the user and/or of the portable unit and the corresponding passwords.

According to a possible embodiment to each user ID or to each portable unit ID a certain list of different available services and or management options can be saved in a memory of the remote service and/or management station 3. In this case, the remote service and/or management station 3 may carry out a process for identifying services and/o management options provided for the corresponding user and/or portable device and for the medical device ID code as shown by the step 731. At step 732 the authorisations for accessing the said services and/or the said management options identified in the previous step 531 are generated and transmitted to the portable and/or wearable device 2.

In response to this the portable and/or wearable unit 2 activates the function keys or interactive selection menus for the authorised service and/or management options as indicated by step 723.

BY using the said function key "buy and download" on the portable and/or wearable unit 2 as indicated by the step 724 access to the online shop is allowed by the remote station 3 as indicated with the step 733.

Download of the desired software upgrade can be started by using the corresponding button on the portable and/or wearable unit 2 as indicated by the step 725. Download is started on the portable and/or wearable device from which the downloaded software is then transferred and installed on the medical device 1 as indicated with the step 511.

It is worth to stress out that the above disclosed examples are in no way limiting nor exhaustive of the possible variants and or of additional features relating to the kind of the services and/or the management operations which may be addressed and carried out. This also apply to the succession of the above disclosed steps and also to specific kind of the communication protocols disclosed.

## Claims

1. A medical diagnostic device remote service and management system is provided the said system comprising:
at least one medical diagnostic apparatus configured to acquire and/or process and/or store medical image data and/or medical non-image data;
at least a remote service and/or management unit including a service and/or management device for carrying out service and/or management tasks on the at least one medical diagnostic apparatus, the service and/or management tasks being carried out automatically or upon request;
a communication link being provided for the at least one medical diagnostic apparatus and the at least one remote service and management unit for exchanging the data for starting a connection between the said at least one medical diagnostic apparatus and the said at least one remote service and/or management unit and for exchanging data for executing the said service tasks and/or management tasks on the said at least one medical diagnostic apparatus;
the said communication link comprising:
a communication device at each of the said medical diagnostic apparatus;
at least one wearable or portable processing unit for a corresponding user or service person, the said at least one processing unit being provided with a communication interface, configured to communicate with the communication device of said at least one medical diagnostic apparatus;
the communication device of the at least one medical diagnostic apparatus and the communication interface of the wearable or portable processing unit being configured to communicate by means of a so called short-range wireless communication protocol;
the wearable or portable processing unit comprising further a communication unit configured to communicate with a communication unit of the at least one remote service and/or management unit,
the said communication unit of the wearable or portable processing unit and of the at least one service and/or management unit being configured to communicate according to a so-called network communication protocol.

2. A medical diagnostic device remote service and management system according to claim 1, in which a short range wireless communication systems and protocols consist in one of the following protocols:
Bluetooth, Wi-Fi, ZigBee, UWB, IR.

3. A medical diagnostic device remote service and management system according to claim 1 or 2 in which the network protocol consist in one protocol chosen from the following list:
HTTP-hyper text transfer protocol (HTTP)-
TCP -Transmission Control Protocol (TCP)-
UDP -User Datagram Protocol (UDP)-
IRC - Internet Relay Chat (IRC)

4. A medical diagnostic device remote service and management system according to one or more of the preceding claims the communication protocols between the wearable and/or portable unit and the remote service and/or management station are configured in such a way that a user ID and a user password are requested for allowing the user of the wearable and/or portable unit to connect the said unit with the remote service and/or management station and to access the possible service and/or management options provided by the said remote service and/or management station.

5. A medical diagnostic device remote service and management system according to one or more of the preceding claims in which data relating to the medical device may be saved in a memory in the control hardware of the medical diagnostic device and be automatically sent or made readable to the wearable and/or portable device, or the said univocal device ID may be in the form of a graphical and or alphanumerical code.

6. A medical diagnostic device remote service and management system according to one or more of the preceding claims in which the wearable and/or portable unit comprises a processor, memories for storing the control software of the wearable or portable unit itself and the software for carrying out the communication processes with the medical diagnostic devices and with the remote service and/or management station, a user interface for inputting selections and or command, a display interface for showing alphanumeric messages and/or images, a camera for acquiring images and optionally also at least one loudspeaker and/or a microphone.

7. A medical diagnostic device remote service and management system according to one or more of the preceding claims in which the wearable and/or portable unit is configured to read an image of the said graphic codes and extract the information coded therein, basically but not only the said univocal device code.

8. A medical diagnostic device remote service and management system according to one or more of the preceding claims in which the wearable and/or portable device is configured by an APP which is saved and installed, and in which App the instruction are coded to the hardware of the said wearable and/or portable device in order to carry out the connections to the medical diagnostic device and to the remote service and/or management station and to access the functions and tasks for servicing and for managing the said medical diagnostic device made available by the remote service and management station for the specific wearable and/or portable unit and/or for the user of the said unit and/or for the medical diagnostic device identified by the univocal device ID.

9. A medical diagnostic device remote service and management method comprising:
Providing at least one medical diagnostic apparatus and configuring it to acquire and/or process and/or store medical image data and/or medical non-image data;
Providing at least a remote service and/or management unit including a service and/or management device for carrying out service and/or management tasks on the at least one medical diagnostic apparatus, the service and/or management tasks being carried out automatically or upon request;
providing a communication link for the at least one medical diagnostic apparatus and the at least one remote service and management unit for exchanging the data for starting a connection between the said at least one medical diagnostic apparatus and the said at least one remote service and/or management unit and for exchanging data for executing the said service tasks and/or management tasks on the said at least one medical diagnostic apparatus;
the said communication link being provided by means of at least one wearable or portable processing unit for a corresponding user or service person, the said at least one processing unit being provided with a communication interface, configured to communicate with the communication device of said at least one medical diagnostic apparatus;
the communication device of the at least one medical diagnostic apparatus and the communication interface of the wearable or portable processing unit being configured to communicate by means of a according to a so called short-range wireless communication protocol;
the wearable or portable processing unit comprising further a communication unit configured to communicate with a communication unit of the at least one remote service and/or management unit,
the said communication unit of the wearable or portable processing unit and of the at least one service and/or management unit being configured to communicate according to a so-called network communication protocol.

10. A method according to claim 9, in which a short range wireless communication systems and protocols consist in one of the following protocols:
Bluetooth, Wi-Fi, ZigBee, UWB, IR.

11. A method according to claim 9 or 10 in which the network protocol consist in one protocol chosen from the following list:.
HTTP -hyper text transfer protocol (HTTP)-
TCP -Transmission Control Protocol (TCP)-
UDP -User Datagram Protocol (UDP)-
IRC - Internet Relay Chat (IRC).

12. A method according to one or more of the preceding claims 9 to 11 in which the communication protocols between the wearable and/or portable unit and the remote service and/or management station are configured in such a way that a user ID and a user password are requested for allowing the user of the wearable and/or portable unit to connect the said unit with the remote service and/or management station and to access the possible service and/or management options provided by the said remote service and/or management station.

13. A method according to one or more of the preceding claims 9 to 12 in which data relating to the medical device is saved in a memory in the control hardware of the medical diagnostic device and be automatically sent or made readable to the wearable and/or portable device, or the said univocal device ID may be in the form of a graphical and or alphanumerical code.

14. A method according to one or more of the preceding claims in which the wearable and/or portable unit is configured to read an image of the said graphic codes and extract the information coded therein, basically but not only the said univocal device code.

15. A method according to one or more of the preceding claims 9 to 14 in which the wearable and/or portable device is configured by an APP which is saved and installed, and in which App the instruction are coded to the hardware of the said wearable and/or portable device in order to carry out the connections to the medical diagnostic device and to the remote service and/or management station and to access the functions and tasks for servicing and for managing the said medical diagnostic device made available by the remote service and management station for the specific wearable and/or portable unit and/or for the user of the said unit and/or for the medical diagnostic device identified by the univocal device ID.
